(19) Europäisches Patentamt
European Patent Office
Office européen des brevets

(11) Publication number: **0 363 829 A2**

(12) # EUROPEAN PATENT APPLICATION

(21) Application number: **89118538.1**

(51) Int. Cl.[5]: **G01N 33/53 , C12P 21/08**

(22) Date of filing: **06.10.89**

(30) Priority: **13.10.88 US 256979**
**18.09.89 US 408689**

(43) Date of publication of application:
**18.04.90 Bulletin 90/16**

(84) Designated Contracting States:
**DE FR GB IT**

(71) Applicant: **MILES INC.**
**1127 Myrtle Street**
**Elkhart,IN 46515(US)**

(72) Inventor: **Baker, Richard E.**
**North 10504 College Circle**
**Spokane, WA 99218(US)**
Inventor: **Dailey, Frank A.**
**East 14217 - 23rd**
**Spokane, WA 99037(US)**
Inventor: **Kordash, Terance R.**
**E. 6312 Jamieson Road Route 3 Box 6468**
**Spokane, WA 99223(US)**
Inventor: **Plunkett, Gregory A.**
**North 14620 Chronicle**
**Mead, WA 99021(US)**
Inventor: **Wyrick, Ronald E.**
**East 4503 Red Road Drive**
**Spokane, WA 99207(US)**

(74) Representative: **Dänner, Klaus, Dr. et al**
**c/o Bayer AG Konzernverwaltung RP Patente Konzern**
**D-5090 Leverkusen 1 Bayerwerk(DE)**

(54) **Predicting sensitivity to injectable radio contrast media with histamine release test.**

(57) The use of a histamine release immunoassay as a predictor of sensitivity (allergic response) to radio contrast media.

EP 0 363 829 A2

# PREDICTING SENSITIVITY TO INJECTABLE RADIO CONTRAST MEDIA WITH A HISTAMINE RELEASE TEST

## Field of the Invention

The invention involves the use of a whole blood histamine release immunoassay as a predictor of sensitivity to injectable radio contrast media.

## Background of the Invention

Retrospective studies show that radio contrast media tests are associated with an 5-10% (U.S. Patent 4,717,657) incidence of reactions. Some studies have indicated reaction rates as high as 14%. These reactions are usually mild consisting of hives, pheripheral swelling, rhinitis, conjunctivitis, etc. However, approximately 1-2% of these reactions can be classified as "acute" and consist of more severe symptoms such as bronchospasm, laryngeal edema and hypotension. One-tenth to 2/10% of these reactions are considered to be quite serious based on the treatment required. Studies also indicate a mortality rate from 1:12,000 to 1:75,000 (Lasser, E.C. et al Pretreatment with Cortosteroids..., New Eng. J. Med. 317, 845-849, 1987).

Once a patient has had an anaphylactoid reaction following the administration of radio contrast media, he is at significantly increased risk of having a subsequent reaction. In fact, the risk increases approximately three-fold. Pretreatment with Benadryl® and Prednisone® decreases the incidence of repeat reactions to less than 5% overall, and most of these reactions are extremely mild. However, currently it is difficult to identify those individuals at increased risk prior to their first contrast media reaction.

Several papers have been written on histamine release in connection with the administration of radio contrast media including but not limited to: Ring et al., Clin. exp. Immunol. (1978), 34, 302-309; Rice et al., J. Allergy Clin. Immunol. (1983), 72:2, 180-186; Salem et al, Am. J. of Med. (1986), 80, 382-384; and Younger et al., J. Allergy Clin. Immunol. (1986), 94-100.

Predictors for adverse reactions to radio contrast media are actively being sought, as see US Patent No. 4,717,657 which describes a method for the activation of complement and measurement of at least one product resulting from complement activation as a predictor of adverse reaction to injection of radio contrast media.

However, there has been no convenient whole blood test yet developed that can predict which patients are likely to have a reaction. Investigators have tried skin tests as well as subconjunctival and intravenous provocative tests to no avail. Subjects have had severe or even fatal reactions to radiographic contrast media infusions following negative provocative tests. In addition, subjects have had severe reactions following the provocative tests themselves. Therefore a simple, convenient in vitro test which can predict sensitivity to intravenous administration of radio contrast media, would provide useful, and sometimes lifesaving, information which is not available at present.

## Summary of the Invention

Invention provides an immunoassay for use in the determination of histamine released in whole blood as a predictor for sensitivity to radio contrast media, which assay is preferably based on an antibody to histamine produced by the cell line having the American Type Culture Collection Accession Number HB 8831.

This immunoassay may be used as a predictive test for determining whether a patient will react adversely when injected intravenously with radio contrast media. Whole blood is tested for histamine release with a histamine release immunoassay employing an antibody produced by a cell line which is a hybrid of a mouse myeloma cell and a spleen cell from a mouse immunized with a histamine immunogen conjugate chosen from the group

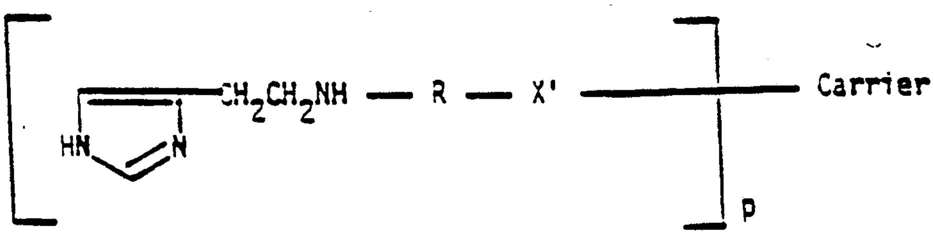

wherein R is an aliphatic chain of from 1 to 10 carbon atoms and X′ is a terminal functional group chosen from amino, carboxyl, thiol and hydroxyl, p is from 1 to 120 and the carrier is an immunogenic carrier material chosen from bovine serum albumin or keyhole limpet hemocyanin. A preferred histamine immunogen conjugate is:

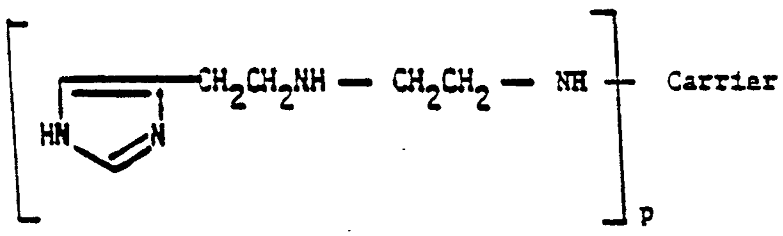

wherein the p is from 1 to 120 and the carrier is bovine serum albumin or keyhole limpet hemocyanin.

Description of the Invention

The use of the histamine release test is described generally in the example following. The patient's whole blood suitable may be used and separation of the cells from the sample is not necessary. Histamine release from the basophils of the patient's whole blood sample can be used to predict when an adverse reaction to any or to a particular radio contrast media may occur. Radio contrast media (RCM) are widely used for diagnostic procedures such as IVP's and Cat Scans. There are many media. Four of the widely used media are Hypaque, Conray, Omnipaque and Isovue.

The histamine release test, described in copending USSN 752,320, filed July 3, 1985 (published January 21, 1987 under European Publication No. 0208953) and commonly assigned with the invention herein, is based on the use of an antibody to an immunogen conjugate of histamine prepared in accordance with that disclosure.

The antibody is produced by a cell line which is a hybrid of a mouse myeloma cell and a spleen cell from a mouse immunized with a histamine immunogen conjugate chosen from the group

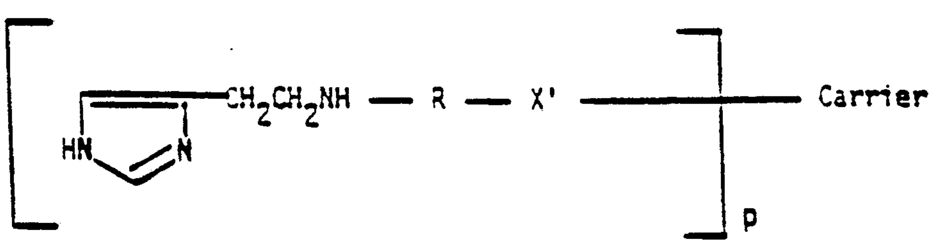

wherein R is an aliphatic chain of from 1 to 10 carbon atoms and X′ is a terminal functional group chosen from amino, carboxyl, thiol and hydroxyl and the carrier is an immunogenic carrier material chosen from bovine serum albumin (BSA) or keyhole limpet hemocyanin. It should be pointed out that as used in the formulae above, "p" represents the number of histamine moieties conjugated to the carrier. The number p is sometimes referred to as the epitopic density of the immunogen and in the usual situation will be on the average from about 1 to about 120, more normally from 1 to about 50. These densities, however, may vary

greatly depending on the particular carrier material used.

A preferred antibody is one raised to the immunogen conjugate

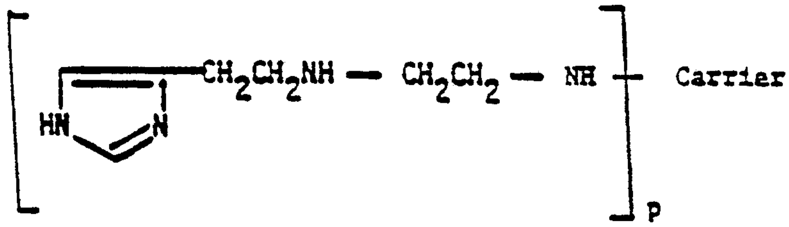

wherein R in $-CH_2CH_2-$, $X'$ is -NH-, p is from 1 to 120 and the carrier material is bovine serum albumin or keyhole limpet hemocyanin. Most preferred is the antibody which is produced by the cell line having the American Type Culture Collection Accession number HB 8831, which was deposited on May 31, 1985 under the Budapest Treaty Convention.

The histamine release test is a competitive system in which free histamine and a histamine-protein conjugate compete for the monoclonal antibody that is highly specific for histamine. The preferred monoclonal has been shown to have little cross reactivity to histidine. A high free histamine concentration in a blood sample will prevent the monoclonal antibody from binding to a histamine-protein conjugate and conversely a low histamine concentration in the patient's blood sample will allow a high concentration of monoclonal antibody to bind to the histamine-protein conjugate. The relative amount of monoclonal antibody binding to the histamine-protein conjugate can be readily estimated by labeling the monoclonal antibody with an enzyme. A particularly convenient format for the test is the use of an enzyme for which the addition of a chromogenic substrate for the enzyme will result in color development if the enzyme and its covalently linked monoclonal antibody are present. A useful combination is peroxidase and one of its chromogenic substrates. However, other enzymes may be used which are well known to those versed in the art. This is the basis of the use of a histamine release immunoassay as a predictor of adverse reaction to the intravenous administration of RCM.

In the first step a histamine-protein conjugate is adsorbed to 94 polystyrene pegs simultaneously. This is accomplished by submerging the pegs into wells of a 96 well microtiter plate (referred to as the adsorption plate) that contain a solution of a histamine-BSA conjugate. Two microtiter wells contain only BSA and are used as negative controls. Following a one hour incubation period at room temperature, the pegs are removed and washed to remove non-adsorbed conjugate.

A second 96 well microtiter plate (referred to as the release plate) containing various dilutions of RCM or histamine is also prepared. Fresh whole blood (patient sample) is diluted in a buffer containing the histamine monoclonal antibody covalently linked to peroxidase, preferably horseradish peroxidase. Equal portions (50 $\mu$l) of diluted blood (54/46:Blood/Diluent (v/v)), per microtiter well, are added to each well, mixed and then incubated for 30 minutes at 37° C. During this incubation period histamine will be released from basophils in the blood sample if the patient is sensitive. Polystyrene pegs, to which histamine-BSA has been adsorbed, are now immersed in the microtiter wells of the release plate for 30 minutes at room temperature. During this time, free histamine and the histamine-BSA conjugate compete for the monoclonal antibody. The polystyrene pegs are then removed and washed.

The relative concentration of the monoclonal antibody-horseradish peroxidase conjugate bound to the histamine-BSA conjugate on the polystyrene pegs is now estimated by immersing the pegs in a third microtiter plate (referred to as the chromogen plate) containing a chromogenic substrate for horseradish peroxidase, 2, 2'-azino-di-(3-ethyl-benzothiazoline)-6-sulfonic acid (ABTS®). High color development means that little free histamine was present in a given blood sample/RCM microtiter well. The converse is true for low color development. Low color development indicates that the patient whose blood is being tested is sensitive to the test RCM. Color development takes 10 to 30 minutes and significant suppression of color development, i.e., high histamine release, is often discernable by eye. Quantitative assessment of histamine release can be made if a Microtiter ELISA Reader is available.

This test requires approximately two hours as compared to previously available histamine release tests which require a day to obtain results. Therefore it is possible to conveniently test patients for adverse reactions to the administration of RCM without endangering them with a provocative in vivo test.

EXAMPLE

Preparation of Histamine-Bovine Serum Albumin Immunogen Conjugate:

75 mg of bovine serum albumin (BSA), 38.4 mg of 1-ethyl-3-(3-dimethylaminopropyl)-carbodiimide and .184 mg of histamine were mixed in a 0.1 molar boric acid solution to a total volume of 4.0 ml (pH maintained at 4-5). After stirring overnight (room temperature) the mixture was dialyzed extensively against phosphate buffered saline. Absorbance at 280 nanometers (nm) was measured and protein concentrations were calculated. Histamine epitope density was determined by scintillation counting using a tritiated histamine tracer, two representative runs having epitope densities of 17 and 53. The conjugates from the two runs were combined and used as described in the assay procedure below.

Histamine Release Assay

The histamine-BSA conjugate prepared as disclosed above was added to 0.05 molar $NaHCO_3$, pH 9.6 to a concentration of 40 µg/ml. 75 µl aliquots of this solution were placed in the wells of a round bottom microtiter tray (referred to as a "coating plate"). Two wells were filled with 75 µl of bovine serum albumin (BSA, 40 µg/ml) in the 0.05 molar $NaHCO_3$ buffer to serve as controls. A lid with 96 pegs (such as NUNC TSP® or Falcon FAST® lids) was then placed on the coating plate and incubated for about one hour at room temperature. The lid with pegs was then removed and washed twice with a total of 50-100 ml of a mixture of 0.05% Tween 20 in distilled water per plate. A second microtiter tray (referred to as the "release plate") was then prepared for the histamine release process as follows.

A histamine standard curve was prepared by placing 10-20 µl of histamine standard solutions in successive duplicate wells. These standard solutions were made such that the final in well histamine concentrations were 333, 111, 37, 12.3 and 4.1 nanomolar (nM) of histamine, respectively in distilled water or Tris ACM diluent (i.e., a buffer of 25 millimolar (mM) Tris (pH 7.6); 0.12 molar NaCl; 5 mM KCl; 0.3 mg/ml human serum albumin; 1 mM $CaCl_2$; and 0.5 mM $MgCl_2$).

The radio contrast media (RCM) to be tested as the allergen was diluted into 8 to 9 serial dilutions using Tris ACM or distilled water as diluent. Aliquots (10-20 µl) from each dilution (not including stock) were placed in successive duplicate wells of the release plate. This process was repeated for each desired RCM to be tested until the finished release plate had 10-20 µl of either histamine standards, RCM dilutions or Tris ACM buffer (for control wells) in all desired wells. The release plate was then ready for use.

Heparinized blood (3.0 ml) from a subject patient was obtained and mixed with 2.56 ml Tris ACM buffer containing a monoclonal anti-histamine antibody-horseradish peroxidase conjugate. This is sufficient for one plate. A monoclonal anti-histamine antibody-horseradish peroxidase conjugate was prepared by the periodate borohydride coupling method described by Paul Nakane, in Immunoassays: Clinical Laboratory Techniques for the 1980's, (Nakamura, R. M., Dito, W. R., and Tucker, E. S., editors), Alan R. Liss, Inc., New York (1978). The monoclonal anti-histamine antibody was prepared as disclosed in copending USSN 752,320 assigned commonly herein. The preferred monoclonal antibody is produced by the cell line having American Type Culture Collection Accession No. HB 8831. A small amount of this antibody-enzyme conjugate was added to the Tris ACM/blood mixture to produce the proper pre-determined conjugate concentration (about 0.5-1.5 µg/ml). Aliquots (50 µl/well) of this mixture were then placed in each well of the release plate and incubated for 30 minutes at 37° C to allow histamine release.

Then, the lid prepared as described above with 96 pegs was placed on the release plate such that each peg was introduced into a well. This configuration was then incubated at room temperature for 30 minutes, after which the lid with the pegs was removed and washed with two 30-50 ml portions of Tween 20/water as described above.

This lid with the pegs was then placed into a third microtiter tray (referred to as the "chromogen plate") to which had been added 50 µl/well of the following solution: 1.6 mg/ml of 2,2′-azino-di-(3--ethyl-benzothiazoline)-6-sulfonic acid (ABTS®); and 10 mM urea peroxide in 0.1 M citrate buffer (pH 4.3).

After a suitable incubation time (about 15 minutes at room temperature) the pegs were removed and the absorbencies (415 nM) of the contents of each well of the chromogen plate were determined using an ELISA reader. Alternatively, an assessment of histamine release can be made by visual inspection particularly where the pegs were first coated in the coating plate with a histamine-protein conjugate, preferably the histamine-bovine serum albumin conjugate prepared as disclosed previously. Percent color

decrease or actual concentrations of released histamine obtained from the standard curve (prepared as described above) can be obtained and/or plotted against the dilution of RCM, if desired. From such plots judgments regarding a patient's sensitivity to the RCM allergen can be made. Histamine release that occurs at dilute RCM level may indicate a high sensitivity and vice versa.

A pilot clinical study was performed with twenty (20) patients who had a history of having reactions to radio contrast media in the past. The patients were tested using four different radio contrast medias: Conray, Hypaque, Omnipaque and Isovue. Each are injectable radio contrast medias which can be used for the same diagnostic procedure or different procedures, such as IVPs, Cat Scans, etc.

The study showed that the histamine release test described above can measure histamine that is released from basophils in patients when their blood is incubated with the radio contrast media. Compared to the control group, there were several patients in the group of previous reactors to the injection of radio contrast media who had significantly more histamine release when their cells were incubated with the radio contrast media Hypaque. This would suggest that the histamine release test described can be used to diagnose (predict) increased sensitivity to radio contrast media. This also would predict that these would be the patients who would have significant reactions to the radio contrast media.

Because of the test's advantages in time and convenience, it may therefore be practical to use histamine release as a predictor of sensitivity (allergy) to radio contrast media.

A clinical study was performed to test the hypothesis that sensitivity (allergy) to radio contrast media could be predicted by the results of the histamine release test described herein. Histamine release from a blood sample from patients who would later be tested with a radio contrast media was measured. The results were later compared with the occurrence of an allergic reaction to the administration of the radio contrast media. Of sixteen patients in the study, one patient had an allergic reaction to the injection of the radio contrast media, Hypaque. That patient was also the patient who showed the highest degree of histamine release when measured with the test.

Many modifications and variations can be made to the practice of the present invention without departing from the spirit and scope thereof which is solely defined by the claims.

## Claims

1. The use of an immunoassay for the determination of histamine release in whole blood as a predictor for sensitivity to radio contrast media, wherein the immunoassay is based on an antibody to histamine produced by the cell line having the American Type Culture Collection Accession Number HB 8831.

2. A predictive test for determining whether a patient will react adversely when injected intravenously with radio contrast media comprising testing whole blood for histamine release with a histamine release immunoassay employing an antibody produced by a cell line which is a hybrid of a mouse myeloma cell and a spleen cell from a mouse immunized with a histamine immunogen conjugate chosen from the group

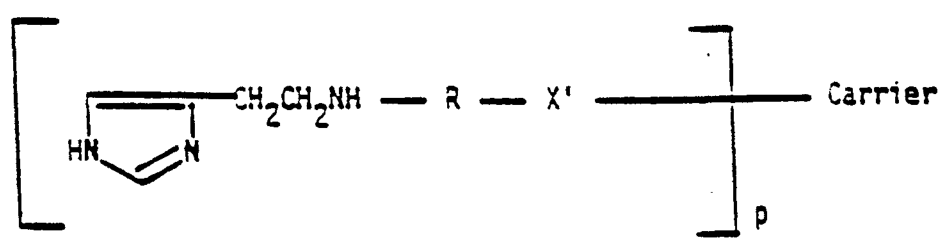

wherein R is an aliphatic chain of from 1 to 10 carbon atoms and X′ is a terminal functional group chosen from amino, carboxyl, thiol and hydroxyl, p is from 1 to 120 and the carrier is an immunogenic carrier material chosen from bovine serum albumin or keyhole limpet hemocyanin.

3. The predictive test of claim 2 wherein the histamine release immunoassay employs an antibody produced by a cell line which is a hybrid of a mouse myeloma cell and a spleen cell from a mouse immunized with the histamine immunogen conjugate:

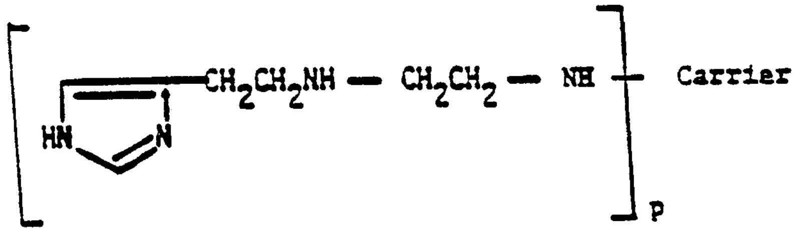

wherein the p is from 1 to 120 and the carrier is bovine serum albumin or keyhole limpet hemocyanin.

4. The predictive test of claim 3 wherein the antibody is produced by the cell line having the American Type Culture Collection Accession Number HB 8831.